# EUROPEAN PATENT APPLICATION

(11) **EP 3 782 981 A1**
(43) Date of publication of application: **24.02.2021**
(21) Application number: 19192468.7
(22) Date of filing: 20.08.2019
(51) Int. Cl.: C07C 303/06, C07C 309/04

(54) **CATALYSTS FOR THE SYNTHESIS OF ALKANESULFONIC ACIDS**

(71) Applicant: BASF SE, 67056 Ludwigshafen am Rhein (DE)
(72) Inventor: KEMPTER, Andreas, 67056 Ludwigshafen (DE); SALA, Oliver, 67056 Ludwigshafen (DE); PIEPENBREIER, Frank, 67056 Ludwigshafen (DE); CHAN, Chee Jian, 67056 Ludwigshafen (DE); BORGMEIER, Frieder, 67056 Ludwigshafen (DE)
(74) Representative: BASF IP Association

(57) **Abstract**

The present invention relates to novel uses of inorganic peroxosulfuric acids as catalysts in the preparation of alkanesulfonic acids from alkanes and sulfur trioxide, methods for the production of alkanesulfonic acids employing said catalysts as well as reaction mixtures comprising said catalysts. The invention particularly relates to the production of methanesulfonic acid from methane and sulfur trioxide employing inorganic peroxosulfuric acids as catalysts.

## Description

The present invention relates to compounds that can be applied as catalysts in the preparation of alkanesulfonic acids from alkanes and sulfur trioxide, methods for the production of alkanesulfonic acids employing said catalysts as well as reaction mixtures comprising said catalysts. The invention particularly relates to the production of methanesulfonic acid from methane and sulfur trioxide or solutions comprising sulfur trioxide employing said catalysts.

Alkanesulfonic acids are organic acids that can reach a similar acid strength as that of inorganic mineral acids, for example, sulfuric acid. However, in contrast to usual mineral acids such as sulfuric and nitric acids, the sulfonic acids are non-oxidizing and do not give off vapors that are harmful to health, as can be observed with hydrochloric and nitric acids. Further, many sulfonic acids, for example, methanesulfonic acid, are biologically degradable. The applications of sulfonic acids are many, for example, in cleaning agents, surfactants, galvanic and electronic industry, as catalysts, and in organic synthesis, pharmaceutical chemistry, for example, as protective groups. The salts of sulfonic acids are employed, for example, as surfactants, for example, sodium dodecylsulfonate, or in the electroplating industry, especially as tin, zinc, silver, lead and indium, but also other metal, alkylsulfonates. Furthermore, organic salts are employed in pharmaceutical chemistry. The very high solubility of alkyl sulfonates plays an important role, in particular. Further, no harmful gases are formed in electrolysis, and the use of toxic compounds, for example, cyanide, which is common in many cases, is dispensed with.

The structurally simplest representative of alkanesulfonic acids is methanesulfonic acid. US 2,493,038 describes the preparation of methanesulfonic acid from SO₃ and methane. US 2005/0070614 describes further methods for preparing methanesulfonic acid, and its application. The methods known in the prior art are in part complicated, cost-intensive, and lead to undesirable products because of the harsh reaction conditions.

The reaction conditions in conventional processes of alkanesulfonic acid production can result in undesirable side products, which even manifest themselves as disturbing inhibitors in the production of alkanesulfonic acids. This may lead to termination of the actual reaction for preparing the alkanesulfonic acid, but also to impurities, formation of side products and poor yields, based on sulfur trioxide and methane.

WO 2007/136425 A2 discloses the use of the compound di(methanesulfonyl) peroxide (DMSP), which must be prepared by a complex electrolysis and, in addition, is a crystallizable highly explosive solid, as an initiator in a reaction in which methanesulfonic acid is formed from sulfur trioxide and methane.

WO 2015/071365 A1 and WO 2015/071455 A1 both describe processes for the sulfonation of alkanes. The main steps are:
1)Synthesis of an initiator/initiator-solution.
2)Preparation of a sulfur trioxide-solution (oleum) by dissolving sulfur trioxide in an inert solvent (e.g. sulfuric acid)
3)Reaction of oleum with the corresponding alkane after or during addition of the initiator/ initiator- solution in a high-pressure-reactor.
4)Quenching of non-reacted starting material
5)Purification (e.g. distillation, crystallization etc.)
6)Recycling of the inert solvent (e.g. sulfuric acid).

According to said prior art, the initiator is particularly prepared by reacting an alkanesulfonic acid R-SO₃H, i.e. the desired product, with hydrogen peroxide in order to form an initiator-precursor R-SO₂-O-OH. Said initiator-precursor is then reacted with SO₃ yielding initiator compounds such as R-SO₂-O-O-SO₃H. The cited prior art therefore requires some amount of the desired product to form an initiator.

It is thus the object of the present invention to provide novel catalysts for the homogeneous catalysis in the preparation of alkanesulfonic acids, especially methanesulfonic acid (MSA). Particularly, it is the object of the invention to provide catalysts that do not require the desired product itself to be present as an initiator-precursor.

In a first embodiment, the object of the present invention is solved by the use of a compound comprising at least one inorganic peroxosulfuric acid or a salt thereof, as a catalyst in the preparation of alkanesulfonic acids from alkanes and sulfur trioxide, especially in the preparation of methanesulfonic acid from methane and sulfur trioxide. Particularly, methane, ethane, propane, butane, isopropane, isobutane or a higher alkane can be reacted with sulfur trioxide to form the corresponding alkanesulfonic acid.

Suprisingly, it has been found that inorganic peroxosulfuric acids show a similar catalytic activity as peroxoacids derived from the desired alkanesulfonic acids or respective inorganic peroxoacids. Therefore, the desired product is not required as a precursor of the catalyst. In principle, any inorganic peroxosulfuric acid can be employed. Such inorganic peroxosulfuric acids can be prepared from their corresponding peroxo- or oxoacids, which are cheaply available from commercial distributors.

According to the invention, the peroxosulfuric acid is used as a catalyst in a condensed-phase homogeneous process. The peroxosulfuric acid catalyst is solved in the same phase as the reactants, i.e., an alkane and sulfur trioxide or mixtures comprising an alkane and sulfur trioxide.

In what follows, the present invention is described in its preferred embodiments. The description is meant to be exemplary and not to limit the scope of the invention.

In a preferred embodiment, the peroxosulfuric acid comprises at least one peroxosulfuric acid of boron, silicon, phosphorus, carbon, nitrogen or sulfur. Any suitable peroxosulfuric acid of said elements can be used. The peroxosulfuric acids are typically derived from the corresponding oxoacid or peroxoacids of the respective element.

Preferably, the peroxosulfuric acid used as a catalyst according to the invention is obtainable by a reaction of the corresponding oxoacid with a peroxide and sulfur trioxide or by reaction of the respective peroxoacid with sulfur trioxide. More preferably, the peroxosulfuric acid is obtainable by a reaction of the corresponding oxoacid with hydrogen peroxide and sulfur trioxide. Without the intention of being bound by theory, the reaction of an oxoacid with hydrogen peroxide and sulfur trioxide can, for example, be described by

EOₐ(OH)_{b}-OH + H₂O₂ +SO₃-> EOₐ(OH)_{b-c}(-O-OSO₃H)_{c}+H₂O. (R1),

with a being an integer from 0 - 4, b being an integer from 0 - 4 and c being an integer from 0 - 4, a≥0 and b-c≥0. In the same manner an oxoacid could form a catalytically active species by reaction with hydrogen peroxide and sulfuric acid or methanesulfonic acid. The resulting solution may therefore contain one catalyst, i.e. catalytically active species, or a mixture of catalytic species resulting from reaction of the oxoacid with hydrogen peroxide and methanesulfonic acid.

Without the intention of being bound by theory, the reaction of a peroxoacid with sulfur trioxide can, for example, be described by

EOₐ(OH)_{b}(-O-OH)_{c} + c SO₃-> EOₐ(OH)_{b}(-O-OSO₃H)_{c}. (R2)

with a being an integer from 0 - 4, b being an integer from 0 - 4 and c being an integer from 0-4.

In a preferred embodiment, the peroxosulfuric acid used according to the invention comprises a polyprotic acid. Particularly, the peroxosulfuric acid may consist of one or more polyprotic acids. Said polyprotic peroxosulfuric acid comprises one or more peroxysulfuryl groups, which can be described by EOₐ(OH)_{b}(-O-O-SO₃-X)_{c}, wherein a being an integer from 0 - 4, b being an integer from 0 - 4 and c being an integer from 0 - 4 and X may be hydrogen and/or an alkaline and/or alkaline-earth metal. More preferably X is hydrogen, lithium, sodium and/or potassium. Most preferably, X is hydrogen.

Preferably, if a polyprotic acid is used, the peroxosulfuric acid comprises one or more hydroxyl groups in addition to the one or more peroxysulfuryl groups. Said hydroxyl groups may be present in form of a salt, i.e., the groups can be described by -O-X, wherein X may be hydrogen, an alkaline metal and/or an alkaline-earth metal. Most preferably X is hydrogen.
If a polyprotic acid is used, the peroxosulfuric acid can comprise one or more peroxy groups in addition to the one or more peroxysulfuryl groups. Said peroxyl groups may be present in form of a salt, i.e., the groups can be described by -O-O-X, wherein X may be hydrogen, CH3, an alkaline metal and/or an alkaline-earth metal. Most preferably X is hydrogen and/or CH3.

The polyprotic acid could be available in monomeric form or in partially condensated from, i.e. as dimer or oligomer or even as polymer. A catalytically active species could be derived from a polyprotic acid in partially condensated form having then the formula EOₐ₋ₓ(OH)_{b-y}(-O-O-SO₃-X)_{c-z} with x, y and z being integer numbers from 1 to 3. For example, in the case of phosphoric acid, the polyprotic acid may be available as HO-P(O2)-OH (monomer) or as polyphosphoric acid, e.g. HO-P(O2)-O-P(O2)-OH or an even higher condensated form HO-P(O2)-O-(P(O2))n-OH with n having values from 1 to 100 or 1 to 10 or 1 to 3, the connections could be in a linear or in a branched chain. Consequently, the catalytic species may be generated by reaction of the polyprotic acid with hydrogenperoxide and SO3 and/or sulfuric acid and/or MSA, or it may be generated by reaction of a partially condensated form of the polyprotic acid with hydrogenperoxide and SO3 and/or sulfuric acid and/or MSA. This also applies to other polyprotic acids described in this text.

In a preferred embodiment of the invention, the reaction product of phosphoric acid (H₃PO₄) with hydrogen peroxide and sulfur trioxide and / or the reaction product of boric acid (H₃BO₃) with hydrogen peroxide and sulfur trioxide and/or the reaction product of potassium peroxomonosulfate (KHSO₅) and sulfur trioxide is used as inorganic peroxoacid according to the invention. Surprisingly, it has been found that said preferred peroxosulfuric acids are particularly suitable as catalyst in the preparation of alkanesulfonic acids from alkanes and sulfur trioxide without negatively influencing yield, conversion, side products and reaction time.

In an alternative embodiment, the object of the invention is solved by the use of a mixture comprising hydrogen peroxide and an inorganic oxoacid together with sulfur trioxide and / or the reaction product of an inorganic peroxoacid with sulfur trioxide as catalyst in the preparation of alkanesulfonic acids from alkanes and sulfur trioxide, especially in the preparation of methanesulfonic acid from methane and sulfur trioxide. Optionally the mixture may comprise a solvent.

In the following, the synthesis of the peroxosulfuric acid is described. The description is meant to be exemplary and not to limit the scope of the invention.
Sulfur trioxide, pure or in a solvent, preferably in sulfuric acid, is mixed with the oxoacid and hydrogen peroxide is added. Alternatively, the oxoacid is mixed with hydrogen peroxide and sulfur trioxide (pure or in a solvent, preferably in sulfuric acid) is added. Oxoacids according to the invention are the oxoacids of boron, silicon, phosphorous, sulfate and carbon.

The solvent may be the target alkanesulfonic acid, or it may be SO3 or sulfuric acid or any other solvent suitable for the reaction or mixtures thereof. It may also be the alkane of the target reaction, i.e. if methanesulfonic acid is the target alkanesulfonic acid the solvent may be methane, or solutions containing said alkane.

In another embodiment, a peroxoacid of boron, silicon, phosphorous, sulfate and carbon are added to sulfur trioxide (pure or in a solvent, preferably in sulfuric acid). Alternatively, sulfur trioxide (pure or in a solvent, preferably in sulfuric acid) is added to a peroxoacid of boron, silicon, phosphorous, sulfate and carbon, optionally in a solvent.
The molar ratio of the oxoacid and sulfur trioxide is 0,1:1 to 5:1. More preferably, the ratio of sulfur trioxide to the oxoacid is at least 1:1, but not higher than the amount of oxo or hydroxyl groups of the respective oxoacid or peroxoacid.

The resulting mixture is used as the initiator. Peroxosulfuric acids according to the invention are preferably the peroxosulfuric acids of boron, silicon, phosphorous, sulfate and carbon.
In an alternative embodiment, the catalyst compound is produced *in situ* in the reaction mixture. Particularly suitable oxoacids comprise oxoacids of boron, silicon, phosphorus, nitrogen, carbon and/or sulfur, suitable peroxoacids comprise the peroxoacids of boron, silicon, phosphorus, nitrogen, carbon and/or sulfur.

Preferably, boric acid (H₃BO₃) and/or phosphoric acid (H₃PO₄) are used in a mixture according to the invention.

In an alternative embodiment, the object of the invention is solved by a process for the preparation of alkanesulfonic acids from alkanes and sulfur trioxide comprising the steps of
i) providing sulfur trioxide;
ii) providing methane
iii) setting a pressure of from 1 to 200 bar;
iv) introducing an inorganic peroxosulfuric acid or a salt thereof;
v) controlling the temperature of the reaction mixture at 0 °C to 100 °C;
vi) reacting the sulfur trioxide with an alkane, especially methane, in a high-pressure autoclave or laboratory reactor;
vii) if necessary purifying the reaction product, for example, by distillation or extraction.

In the procedure described above as one possible embodiment of the invention the order especially of steps iii) to v) could be changed.

In an alternative embodiment the target conditions could be approached by stepwise adjusting synthesis conditions, e.g. feeding the raw materials (steps i) and ii)) or setting target pressure and temperature in the reaction sequence. This may become helpful when trying to avoid that the system overswings certain target conditions, e.g. that the temperature rises too much or alternatively that the pressure rises too much or even that pressure and temperature overswing. That may happen e.g. when the raw materials are added too quickly or upon addition of the starter in step iv).

The inventive process differs from similar processes from the prior art in that in step iv) an inorganic peroxosulfuric acid is added as catalyst. Said peroxosulfuric acid corresponds to the abovementioned peroxosulfuric acids which may be used as catalysts.

Sulfur trioxide may be provided in the form of oleum, i.e., a solution of sulfur trioxide in sulfuric acid. Instead of oleum also pure sulfur trioxide can be employed. This avoids the preparation of sulfur trioxide solutions. The reaction conditions are here without added solvents. Further, non-reacted sulfur trioxide can evaporate, avoiding the necessity of quenching it.

In a further embodiment, sulfur trioxide is used in a form of oleum with a trioxide content e.g. of 24% (w/w), or 32% (w/w), or 50 % (w/w), or 65 % (w/w), or 70% (w/w) or 80% (w/w) or more. Of course, any concentration in between this concentration window could be used as well. Even pure sulfur trioxide (100 % (w/w) sulfur trioxide) may be used.

A further embodiment of this invention may be the use of pure sulfur trioxide as mentioned above. As contrary to the prior art, a circulation of solvent is not necessary, alkanes comprising higher amounts of impurities compared to the prior art can be used. Impurities usually are enriched in the solvent leading to a reduced yield of alkanesulfonic acids. By avoiding solvents and thus a circulation of them, impurities originating from the alkanes are not negatively influencing the production of alkanesulfonic acids when pure sulfur trioxide is employed.

Sulfur trioxide is reacted with an alkane in a reactor. For alkanes with a low boiling point, the use of a high-pressure reactor is necessary. For pentane and higher alkanes, a common laboratory reactor is sufficient. In the case of gaseous alkanes, for example, methane, a pressure of 1 to 200 bar gas pressure is set. Preferably, a pressure of 30 to 150 bar is set. Particularly preferred is a pressure of 50 to 120 bar or 60 to 110 bar.

The temperature may be set to 10°C to 100°C, preferably to 40°C to 80°C and particularly preferably to temperatures between 45°C and 65°C.

Subsequently, the peroxosulfuric acid catalyst according to the present invention is added. The catalyst may be provided in pure form or solved in a suitable solvent. Preferably, the initial molar ratio between the catalyst and SO₃ is in the range of 1:50 to 1:10000, more preferably 1:100 to 1:500, particularly 1:150. The catalyst may be provided in a solvent, particularly in sulfuric acid.

After the reaction has taken place, the reaction mixture contains essentially the respective alkanesulfonic acid, especially methanesulfonic acid, as well as sulfuric acid. This mixture of alkanesulfonic acid, especially methanesulfonic acid (MSA), and H₂SO₄ might afterwards be used as it is. The combination of an alkanesulfonic acid, especially methanesulfonic acid, and sulfuric acid provides a strong acid in which even gold might be dissoluted enabling different fields of technical applicability.

Alternatively, the alkanesulfonic acid, especially MSA, might be separated i.e. the method of the invention comprises the optional step of the purifying the reaction product, which might be done by distillation or extraction.

But also alkanesulfonic acids, and specially methanesulfonic acids, might be used in different technical fields, i.e. as cleaning agent (cleaning comprising the area of cleaning and caring, home care as well as industrial and institutional cleaning of hard and soft surfaces, i.e. in dishwashing, commercial laundry, cleaning and sanitation, vehicle and transportation care, concrete cleaning, membrane cleaning, and others), for regeneration of ion exchange resins, in galvanic proceedings, in the area of oil, gas, mining, treatment of metals and/or their surfaces, in different areas of pharmaceutical, chemical and argro-chemical industry or in the production of biodiesel. MSA might also be used in galvanization process of plastics, the broad area of batteries, such as lead battery recycling and recycling in general, such as metal recycling, as well as borane generation are further possible areas of application.

In an alternative embodiment, the object of the invention is solved by a process for the preparation of alkanesulfonic acids from alkanes and sulfur trioxide comprising the steps of
i) providing sulfur trioxide;
ii) providing methane
iii) setting a pressure of from 1 to 200 bar;
iv) introducing an inorganic peroxoacid or a salt thereof and sulfur trioxide, wherein the peroxoacid and the sulfur trioxide are introduced sequentially or simultaneously;
v) controlling the temperature of the reaction mixture at 0 °C to 100 °C;
vi) reacting the sulfur trioxide with an alkane, especially methane, in a high-pressure autoclave or laboratory reactor;
vii) if necessary purifying the reaction product, for example, by distillation or extraction.

In the procedure described above as one possible embodiment of the invention the order especially of steps iii) to v) could be changed.

The process according to this embodiment of the invention differs from the aforementioned embodiment of the inventive process in that the catalyst is employed by introducing a peroxoacid and sulfur trioxide rather than a peroxosulfuric acid. The peroxosulfuric acid is thus formed *in situ* in the autoclave or laboratory reactor in which the reaction of sulfur trioxide and the alkane takes place.
Any suitable peroxoacid which can be used in a catalyst mixture in the preparation of alkanesulfonic acids as defined above can be employed.

Particularly suitable peroxoacids comprise peroxoacids of boron, silicon, phosphorus and/or sulfur. The peroxoacid may be a monoprotic or a polyprotic acid. Particularly, if a polyprotic acid is used, only a part, especially only one, of the peroxyl groups may be replaced by peroxysulfuryl groups upon the reaction with sulfur trioxide or with sulfuric acid or with methanesulfonic acid. Preferably, other peroxoacids of boron and phosphorous and / or the reaction products of boric acid and or phosphoric acid with hydrogen peroxide are employed as peroxo acids.

Both the peroxoacid and sulfur trioxide are added to the reactor. They can be added in a mixture, optionally with a solvent. Suitable solvents comprise sulfuric acid or a liquid alkanesulfonic acid, e.g. methanesulfonic acid. The peroxoacid and sulfur trioxide may also be added separately or simultaneously. Sulfur trioxide might be added in pure form or as oleum. If both compounds are added separately, each may optionally be solved in a solvent, for example sulfuric acid. In yet another alternative, both compounds may be added sequentially, wherein each compound may be added as the first or the second compound.

Preferably, the peroxoacid and sulfur trioxide are added in a molar ratio of 1:5 to 5:1, more preferably in a molar ratio of 1:2 to 2:1, most preferably in a molar ratio of 1:1.

The initial molar ratio between the peroxoacid and the SO₃ in the formation of the peroxosulfuric acid is preferably in the range of 1:50 to 1:10000, more preferably in the range of 1:100 to 1:500.

In an alternative embodiment, the object of the invention is solved by a mixture comprising an alkane, sulfur trioxide, aninorganic peroxosulfuric acid and optionally a solvent. The inventive mixture is capable of producing an alkanesulfonic acid. Particularly, if the mixture is set at a pressure of 1 to 100 bar and held at a temperature of 0 to 100 °C, an alkanesulfonic can be produced in an efficient way. The inorganic peroxosulfuric acid acts as a catalyst.

In a preferred embodiment, the alkane is methane. Such a preferred mixture is capable of forming methanesulfonic acid.

In an alternative embodiment, the object of the invention is solved by a mixture comprising an alkane, sulfur trioxide, an inorganic oxoacid, hydrogen peroxide and optionally a solvent, wherein the inorganic oxoacid is capable of forming an inorganic peroxosulfuric acid. The inventive mixture is capable of producing an alkanesulfonic acid. Particularly, if the mixture is set at a pressure of 1 to 100 bar and held at a temperature of 0 to 100 °C, an alkanesulfonic can be produced in an efficient way. The inorganic oxoacid and the hydrogen peroxide react to *in situ* form an inorganic peroxosulfuric acid, which is capable of acting as catalyst.

In a preferred embodiment, the alkane is methane. Such a preferred mixture is capable of forming methanesulfonic acid.

## Claims

1. Use of a compound comprising at least one inorganic peroxosulfuric acid or a salt thereof as a catalyst in the preparation of alkanesulfonic acids from alkanes and sulfur trioxide, especially in the preparation of methanesulfonic acid from methane and sulfur trioxide.

2. Use according to claim 1, wherein the peroxosulfuric acid comprises at least one peroxosulfuric acid of boron, silicon, phosphorus or sulfur.

3. Use according to claim 1 or 2, wherein the peroxosulfuric acid is obtainable by reaction of a peroxoacid with sulfur trioxide.

4. Use according to claim 1 or 2, wherein the peroxosulfuric acid is obtainable by reaction of an oxoacid with a peroxide and sulfur trioxide, especially with hydrogen peroxide and sulfur trioxide.

5. Use according to one or more of claims 1 to 4, wherein the peroxosulfuricacid comprises, especially consists of, a polyprotic acid comprising one or more peroxysulfuryl groups -O-O-SO₃X, wherein X is H, Li, Na and/or K.

6. Use according to claim 5, wherein the polyprotic acid further comprises one or more groups -O-X, wherein X is H, Li, Na and/or K.

7. Use according to claim 5 or 6, wherein the polyprotic acid comprises one or more groups -O-O-X, wherein X is H, Li, Na and/or K.

8. Use according to any one of claims 1 to 7, wherein the peroxosulfuric acid comprises the reaction product of phosphoric acid with hydrogen peroxide and sulfur trioxide, the reaction product of boric acid with hydrogen peroxide and sulfur trioxide and/or potassium peroxomonosulfate and sulfur trioxide.

9. Use according to any one of claims 1 to 8, wherein the peroxosulfuric acid comprises the reaction product of a peroxoacid of phosphorous with sulfur trioxide, the reaction product of a peroxoacid of boron with sulfur trioxide and/or potassium peroxomonosulfate and sulfur trioxide.

10. Use according to any one of claims 1 to 9, wherein sulfur trioxide, pure or in a solvent, preferably in sulfuric acid, is mixed with the oxoacid, preferably with an oxoacid of boron, silicon, phosphorous, sulfate or carbon, and hydrogen peroxide is added.

11. Use according to any one of claims 1 to 10, wherein the oxoacid is mixed with hydrogen peroxide and sulfur trioxide (pure or in a solvent, preferably in sulfuric acid) is added, wherein the oxoacid preferably is an oxoacid of boron, silicon, phosphorous, sulfate or carbon.

12. Use of a mixture comprising hydrogen peroxide, an inorganic oxoacid, sulfur trioxide and optionally a solvent as catalyst in the preparation of alkanesulfonic acids from alkanes and sulfur trioxide, especially in the preparation of methanesulfonic acid from methane and sulfur trioxide.

13. Use of a mixture according to claim 12, wherein the mixture comprises an inorganic peroxoacid, sulfur trioxide and optionally a solvent as catalyst in the preparation of alkanesulfonic acids from alkanes and sulfur trioxide, especially in the preparation of methanesulfonic acid from methane and sulfur trioxide.

14. Use according to any of claims 1 to 13, wherein the peroxosulfuric acid is a monoprotic acid or a polyprotic acid, especially a polyprotic acid selected from the group of polyprotic peroxoacids of boron, silicon, phosphorus and/or sulfur, boric acid and/or phosphoric acid.

15. Process for the preparation of alkanesulfonic acids from alkanes and sulfur trioxide comprising the steps of
i) providing sulfur trioxide;
ii) providing methane
iii) setting a pressure of from 1 to 200 bar;
iv) introducing an inorganic peroxosulfuric acid or a salt thereof, or introducing an inorganic peroxoacid or a salt thereof and sulfur trioxide, wherein the peroxoacid and the sulfur trioxide are introduced sequentially or simultaneously;
v) controlling the temperature of the reaction mixture at 0 °C to 100 °C;
vi) reacting the sulfur trioxide with an alkane, especially methane, in a high-pressure autoclave or laboratory reactor;
vii) if necessary purifying the reaction product, for example, by distillation or extraction.

16. Process according to claim 15, wherein the alkane is methane and the alkanesulfonic acid is methanesulfonic acid.

17. A mixture comprising an alkane, sulfur trioxide, an inorganic peroxosulfuric acid and optionally a solvent.

18. A mixture comprising an alkane, sulfur trioxide, an inorganic peroxoacid, sulfur trioxide and optionally a solvent, wherein the peroxoacid is capable of forming an inorganic peroxosulfuric acid.

19. A mixture according to claim 17 or 18, wherein the alkane is methane.
